(19) Europäisches Patentamt
European Patent Office
Office européen des brevets

(11) **EP 3 329 837 A1**

(12) **EUROPEAN PATENT APPLICATION**

(43) Date of publication:
06.06.2018 Bulletin 2018/23

(51) Int Cl.:
*A61B 3/032* (2006.01)    *A61B 3/02* (2006.01)

(21) Application number: 16002585.4

(22) Date of filing: 02.12.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: **Carl Zeiss Vision International GmbH**
**73430 Aalen (DE)**

(72) Inventors:
• **Ohlendorf, Arne**
 **72072 Tübingen (DE)**
• **Wahl, Siegfried**
 **73072 Donzdorf (DE)**
• **Ivanov, Iliya**
 **72076 Tübingen (DE)**
• **Leube, Alexander**
 **72072 Tübingen (DE)**

(74) Representative: **Schwenkenbecher, Jan Michael Pateris**
**Patentanwälte PartmbB**
**Markgrafenstraße 22**
**10117 Berlin (DE)**

(54) **METHOD FOR PRESENTATION OF NEW OPTOTYPES, WHICH DISCRIMINATION REQUIRE GLOBAL INFORMATION INTEGRATION BY THE HUMAN VISUAL SYSTEM, TEST SYSTEM FOR MEASURING REFRACTIVE ERROR AND CONTRAST SENSITIVITY, AND COMPUTER PROGRAM PRODUCT**

(57)    A method for displaying optotype representations to assess at least one of a refractive error or a contrast sensitivity of the visual system of a test person comprises providing (11) a plurality of optotypes, comprising a base pattern and one or more modulated versions of said base pattern, wherein a contour of said base pattern is defined by a closed curve and said one or more modulated versions of said base pattern have contours being defined by modulated versions of said closed curve; providing (12) a test system (30) for measuring at least one of a refractive error or a contrast sensitivity of the visual system of a test person; and displaying (13) representations of said plurality of optotypes via a display device (31) of said test system (30).

Further, an optotype representation and its use in a test of visual system, a test system and a computer program product are provided.

FIG 2F

EP 3 329 837 A1

## Description

## Field

The present disclosure in general relates to a class of optotypes for the assessment of the visual system of a test person, such as the assessment of refractive errors, the contrast sensitivity and the neural transfer function of the eye. More particularly, the present disclosure first relates to a method for displaying optotype representations to assess at least the refractive error or the contrast sensitivity of the visual system of a test person. Second, it also relates to an optotype representation and a use of an optotype representation in a test of the visual system of a test person. Further, it relates to a test system for measuring at least one of a refractive error or a contrast sensitivity of the visual system of a test person, and to a computer program product.

## Background

The "visual system" of a person is the part of the central nervous system dedicated to the processing of the visual information. It refers to the whole chain for processing the visual impressions, i.e. the optics of the eye, the retina, the optic nerves and those regions of the brain, here referred to as the visual cortex. These complex structures are involved in detecting and interpreting information from light received by the eyes to build a representation of the surrounding environment. In order to correct optical aberrations of the visual system, characteristics of the visual system are measured, such as the refraction of the eye and the contrast sensitivity. For this task, optoytpes i.e. figures or letters of different sizes have been defined and used in testing the visual acuity.

Currently available optotypes only rely on resolution acuity to measure subjective refractive errors or contrast sensitivity. In case of the assessment of refractive errors, current optotypes represent a defined spatial resolution depending on the distance of the observer to the optotype and the size of the currently used optotypes rely on a resolution mask. Therefore, mostly optical transfer functions are considered during the process of subjective refraction testing, without sufficiently taking into account the further processing of the visual information, received by the eye(s), by areas of the visual cortex.

The most frequently used optotype is the so called Landolt C, where the stroke and the gap widths are equal to the 1/5 of the letter diameter. Any other used optotype has to be comparable with the Landolt C, both in its size and its perceptibility, in order to enable comparison of results. These currently used optotypes are typically used under high or low contrast conditions and the task for the test person is to correctly identify the displayed optotype or its orientation, such as the position of the gap of presented Landolt C optotypes.

However, since these optotypes are used to gain the best subjective correction of refractive errors following the rule "highest visual acuity with highest plus correction", the correction of the refractive error may not be perceived as the best possible result, as it relies on optotypes, such as the Landolt C, which represent the response of the visual system only to high spatial frequencies.

Furthermore, for measuring the contrast sensitivity or the neural contrast transfer function of the eye, contrast gratings (e.g. Gabor patches) are used. The measurement of the contrast sensitivity function is usually performed by displaying, at selected spatial frequencies, differently oriented Gabor patches, and reducing a contrast between the Gabor patch and the background until the test person fails to determine the correct orientation of the displayed Gabor patch, thereby defining the contrast sensitivity threshold value at the corresponding spatial frequency.

The modulation transfer function (MTF) of an eye of a test person, which describes how the optics of the eye transfer contrast received from a scenery onto the retina of the eye, can be determined by using wavefront aberrometry at the Fourier transform of wavefront aberrations to the frequency domain. The neural part of the perception of the gratings can then be calculated from the measured modulation transfer function (MTF) and the contrast sensitivity function (CSF). A neural transfer function (NTF) can be calculated as NTF = CSF / MTF.

However, with these gratings the perception of contrast, i.e. the difference in luminance or color between objects in a same field of view, can be influenced by an imperfect correction of habitual refractive error(s) or aberration(s) of the eye of the test person. For example, the perception of contrast is changed by spherical as well as astigmatic errors. Additionally, currently known solutions, such as the use of Gabor patches, do not efficiently stimulate higher cortical areas of the visual cortex, where a global integration of local features, e.g. oriented edges that are extracted by optical, retinal and cortical early-stage processes allows to identify complex shapes, e.g. triangles, from local image features.

Investigation of the capabilities of the visual cortex of the brain has revealed that higher regions of the visual cortex perform a post-processing of the vision impressions received by the retina and processed by earlier regions of the visual cortex, which perform, e.g., an extraction of basic local features. Higher regions of the visual cortex then, for example, carry out a global integration of the local features. Only such a post-processing could explain visual perception in a hyper-acuity range, where the perceived spatial resolution is even higher than supported by the density of the cones on the retina of the eye. For example, it has been shown that suitable stimuli for investigating global integration of local features in higher cortical areas, are radial frequency patterns, as described in Wilkinson, Frances, Hugh R. Wilson and Claudine Habak, "Detection and recognition of radial frequency patterns", Vision research 38.22 (1998): 3555-3568. The physiological origins of the different global shape processing stages are not yet well understood but begin in visual areas V1 and V2 of the visual cortex with local orientation and curvature extraction, progress through visual area V4 with concentric and curvature-based neural responses, and are thought to become invariant to position and spatial scale within the higher stages of the inferotemporal cortex.

## Summary

It is an objective of the present invention to improve the determination of the characteristics of the visual system of a test person. The measured characteristics are used for correcting individual visual defects in such a way that they better reflect the individual visual perception being influenced by the processing of the visual impressions by the visual cortex of the brain.

This objective is solved by a method for displaying optotype representations to assess at least one of the refractive error or the contrast sensitivity of the visual system of a test person as stated in claim 1. An optotype representation displayed according to the method is disclosed in claim 9, while a use of such optotype representation in a test of the visual system of a test person is disclosed in claim 10. A corresponding test system for measuring at least one of the refractive errors or the contrast sensitivity of the visual system of a test person and a computer program product are disclosed in claims 14 and 15, respectively. Advantageous additional embodiments of the invention are disclosed in the respective dependent claims.

According to an aspect of the invention, a method for displaying optotype representations to assess at least one of a refractive error or a contrast sensitivity of the visual system of a test person comprises at least the following steps:

- providing a plurality of optotypes, comprising a base pattern and one or more modulated versions of said base pattern, wherein a contour of said base pattern is defined by a closed curve and said one or more modulated versions of said base pattern have contours being defined by modulated versions of said closed curve;

- providing a test system for measuring at least one of a refractive error or a contrast sensitivity of the visual system of a test person; and

- displaying representations of said plurality of optotypes via a display device of said test system.

Optotypes are symbols for testing vision. Commonly used standardized optotypes are usually letters, numbers, or geometric symbols such as the often used Landolt C. An optotype representation refers to an optotype as actually displayed with a certain set of parameters, e.g. size, contrast, on a display device, such as a computer monitor or other display panel used in the test system. As another example, a display device may also refer to a chart board displaying printed optotypes.

A contour which is defined by a closed curve can theoretically be completely drawn by a single stroke with a given stroke width and cross-sectional luminance profile without drawing a section of the curve twice and where the end of the curve is identical to its beginning. For example, contours of letters "C" or "E" cannot be defined by a closed curve, whereas contours of letter "O", a circle or an ellipse can be defined by a closed curve.

A test system for measuring at least one of a refractive error or a contrast sensitivity comprises, for example, the display device and a processing device for processing user input provided by the user in response to displayed optotype representations, and, e.g., for providing the optotypes to the display device. Depending on the test carried out, additional means may be provided as part of the test system. For example, for a subjective refraction test, the test system may comprise a set of trial lenses and means for changing the lenses in response to the perception of the presented optotypes by the test person, e.g. a digital phoropter. As another example, for measuring contrast sensitivity, the test system may, e.g., provide means for changing the contrast between the displayed optotype representations and the background until the test person fails to discriminate between displayed optotype representations.

The solution according to the aspect of the invention allows to modify test systems for measuring the refractive error or the contrast sensitivity of the eye in that a subjective refraction test or a measurement of the contrast sensitivity function can be carried out using representations of optotypes that stimulate areas of the higher visual cortex.

The shown solution has the effect that the human visual system must integrate information along the full contours, since only by employing shape discrimination capabilities of the higher visual cortex a discrimination between variations of the modified versions and the base pattern is possible. This process demands global integration of the local features such as oriented edges extracted by optical, retinal and cortical early-stage processes. As a consequence, the results of tests of the visual system will better reflect the characteristics of the visual system as perceived by the individual test person and can, therefore, be used for providing more personalized, optimal correction of aberrations, for example, the refractive errors of the eye.

In one embodiment said closed curve is a circle having a radius $R_0$ and said modulated versions of said closed curve are radially modulated versions of said circle, i.e. a spatial modulation, which may or may not be a modulation by a periodic function, is imposed along the radius of the circle. This advantageously takes into account that the healthy human visual system is able to perform in a hyper-acuity range when detecting deviations from circularity. That is, it is able to detect shape differences smaller than the average diameter of cones in the fovea of the eye, which otherwise are limiting factors in visual acuity testing using common optotypes.

In an example embodiment, said radially modulated versions are sinusoidally modulated versions of said circle, where a sinusoidal modulation is applied to the radius $R_0$ of this reference circle base pattern given by the following formula:

$$ r(\theta) = R_0[1 + A(\theta) \cdot sin(\omega \cdot \theta + \varphi)], $$

where $r$ (radius) and $\theta$ (polar angle) are polar coordinates of the radially modulated versions of said circle, i.e. $r$ defines the radius of the modulated or deformed version of the circle at polar angle $\theta$, $R_0$ is the radius of the circle being modulated, $A(\theta)$ is a radial amplitude or spatial modulation amplitude, i.e the amplitude defining the maximum of the radial modulation imposed on the circle. Further, $\omega$ is a radial modulation frequency or radial frequency, and $\varphi$ is a modulation phase, i.e. the angular phase of the modulated version of the circle determining its orientation. The radial frequency determines the number of cycles in the pattern per 360 deg and the amplitude defines the modulation of the cycle. Conveniently, the radial amplitude $A(\theta)$ is not permitted to exceed 1.0 (expressed as a proportion of the radius $R_0$) and the radial frequency $\omega$ is always set as an integer value. The resulting modulated versions of the circular base pattern exhibit defined numbers of lobes, are closed and never cross themselves and, therefore, are perceived as characteristics of object boundaries. In G. Loffler "Perception of contours and shapes: Low and intermediate stage mechanisms", Vision Research, 48 (2008) 2106-2127 for example, it was demonstrated that these modulated patterns or radial frequency (RF) patterns have a very high sensitivity when used as stimuli for shape discrimination tests, since shape discrimination features of the higher visual cortex are stimulated when the visual system processes RF patterns.

However, it is also possible to test shape discrimination or deviations from a shape with other patterns, such as quadratic, rectangular or ellipsoidal shapes, with three-dimensional shapes, four-dimensional shapes (3D + time and/or motion), or the two-dimensional shapes with changes over time and/or motion. Potentially, all forms of shapes the human brain is trained during life can be considered for a potential pattern and many thinkable deviations (symmetrical or unsymmetrical), such as deviations like a spline or several splines or small deviations of only a pixel or a few pixels.

In an embodiment the contour of the base pattern (and thereby the contours of the modulated versions of the base pattern) exhibits a cross-sectional luminance profile where a light region is bordered on an inside and on an outside by a dark region. For example, if the background used for the optotype representation is chosen to be of grey luminance, a light region is displayed with a higher luminance value than the background, whereas a dark region is displayed with a luminance value below that of the light region and preferably below that of the background. The dark region may, for example, be a halo region. In a preferred embodiment, the cross-sectional luminance profile is defined by a radial fourth derivative of a Gaussian function, resulting in a pattern that is bandwidth limited in spatial frequency. The spatial frequency of the displayed optotype representations can easily be varied as part of a visual test and the cross-sectional luminance profile will remain bandwidth limited in spatial frequency.

In one embodiment said displaying comprises displaying said plurality of optotypes spatially adapted to correspond to a size of a standardized optotype, for example standardized according to DIN EN ISO 8596, where, e.g., the Landolt C is defined. The actual size will depend on the distance between the display device and the test person. The size should correspond to the "standard size" in order to represent a corresponding same relationship between spatial frequency and visual acuity to allow comparison with measurement results from tests employing representations of, e.g., the Landolt C.

In one embodiment said displaying, i.e. displaying representations of said plurality of optotypes via a display device of said test system, comprises changing a contrast between said optotype representations and a background on which said optotype representations are displayed. While state of the art optotypes such as the Landolt C which are used for the measurement of the visual acuity, during the process of subjective refraction, are usually used with a high contrast (or sometimes alternatively with a low contrast) and do not incorporate the effect of the early visual system and/or higher visual cortices (i.e. the higher visual centers), the patterns provided according to the described method can be used, for example, during a subjective refraction test under low, i.e. less than 100%, and high contrast conditions, high and low luminance, thereby enabling photopic, scotopic and mesopic vision tests. If optotypes are used to measure the contrast sensitivity of the eye, the contrast of an optotype is typically changed in order to find the smallest detection threshold. In contrast to the current state-of-the-art optotypes, detection and discrimination between these new optotypes is impossible without visual information processing in areas of the higher visual centers (e.g. visual cortex) and can easily be measured under different contrast conditions. The contrast can be conveniently changed externally, without a need for re-generating the optotypes.

In one embodiment said displaying comprises at least one of displaying said optotype representations as moving objects or displaying said optotype representations with changing contours over time. The perception of motion of the pattern or within the pattern (by change of the contour over time) requires neural processing in the visual cortex of the test person, and results of a visual test comprising a motion parameter will therefore contribute to personalizing the test results to the individual aberrations of the visual system of the test person.

In one embodiment displaying said optotype representations comprises displaying one or more of said optotype representations at least partly using one or more colors. This may activate different pathways in the visual cortex than with the only luminance defined patterns and may be used in a visual test to isolate, and respectively stimulate, predominantly specific dedicated higher cortical areas. Black, white and grey are not considered colors in this context.

According to another aspect of the invention, an optotype representation is disclosed that is displayed according to one of the embodiments of the method described above. The optotype representation implements the advantages and characteristics of the claimed method for displaying such optotype representations. Further according to another aspect of the invention an optotype representation as disclosed above is used in a test of the visual system of a test person. For example, when performing the test of the visual system using radial frequency patterns as optotype representations, the spatial frequency (visual acuity = highest visible spatial frequency) and the referred viewing distance between the displayed optotype representation and the test person, the radial frequency used for modulating the circular base pattern, a contrast and a size of the presented optotype representations is provided.

In one embodiment said test of the visual system of a test person comprises a subjective refraction test. With a subjective refraction test, a refractive error of the visual system of the test person is determined, and the best (combination of) lenses that will provide the best corrected visual acuity. The test relies on cooperation of the test person, who is requested to identify displayed optotype representations, e.g., through different lenses trying to correct the refractive error. According to the embodiment, the subjective refraction test is carried out using optotype representations as described above instead of standard optotypes such as the Landolt C. The contrast can be changed from externally and can be set to 100% or less, depending on the task and the steps within the subjective measurements of the refractive error. Due to the stimulation of higher areas of the visual cortex of the test person, the measured refractive error will correspond better to the refraction as actually perceived by the test person and will not only reflect the physical aberration of the optical parts of the eye, allowing a correction better adapted to the actual perception of the aberration by the test person.

In another embodiment said test of the visual system of the test person comprises a contrast sensitivity test. Instead of requesting the test person to identify an orientation of a grating, e.g. a Gabor patch, one or more modulated versions of the base pattern, e.g. radial frequency patterns, i.e. radially modulated versions of a circle, are presented to the test person, displayed at different spatial frequencies, and under varying contrast conditions, requesting the test person to discriminate between modulated versions and the base pattern. The determination of a threshold where shape discrimination under certain contrast conditions for said new test patterns at different spatial frequencies defines the contrast sensitivity function (CSF) of the visual system, now taking better into account the global integration within the higher areas of the visual cortex.

In one embodiment said test of the visual system further comprises a measurement of a modulation transfer function (MTF) of the visual system of the test person and a determination of a neural transfer function (NTF) based on said contrast sensitivity test and said measured modulation transfer function. To reconstruct the modulation transfer function (MTF) of an eye of the test person, wavefront aberrations, e.g. described by Zernike polynomials, can be used that are normally measured using wavefront aberrometry. Nevertheless, any other description, e.g. using Bessel polynomials, Jacobi polynomials, Legendre polynomials, other orthogonal polynomials as well as mathematical descriptions of aberrations (such as Splines or any other polynomials), could be used. Since these polynomials or mathematical descriptions are normally measured using wavefront aberrometry, also any other objective or subjective method that is used to assess habitual refractive errors, can be used.

To assess the CSF, the spatial resolution of the currently used optotype representations is provided. The contrast of the optotype can be changed from externally and can be varied form 100%, depending on the task and, e.g., the form of a staircase procedure that can be used to measure the contrast sensitivity.

However, the transfer function between object contrast and perceived contrast in the human visual systems is not fully described by the optical transfer properties of the eye. Therefore, the neural contrast transfer function (NTF) is considered that describes the modulations in the perception of contrast for the retina-brain-system. The NTF can be calculated using the following equation:

$$NTF = CSF / MTF.$$

Due to the measurement of the CSF based on the presented optotype representations that stimulate higher areas of the visual cortex, the calculated neural transfer function provides an improved transfer function describing a relationship between object contrast and contrast as perceived via the higher visual system areas of the test person.

The assessment of the individual neural transfer function of the visual system of the test person using the new class of optotypes can be used to derive individual image quality metrics to optimize the prediction of visual performance, like refraction, depth of focus etc.

Further the assessment of the individual neural transfer function allows to achieve best individual performance for spectacles, contact lenses, or intraocular lenses, such that a better glare/halo outcome can be achieved and less refractive surprises are encountered. This may also be useful, e.g., for data glasses or virtual reality devices.

Further, by comparison with results from tests using standard optotypes, it is now possible to distinguish between optical and neuronal effects, e.g. for screening or adaptation prediction. Based on the possibility now to distinguish between optical and neuronal effects, an early stage diagnosis possibility becomes available for ocular as well as neuronal diseases, e.g. Alzheimer and Parkinson, or for monitoring treatment success. Diagnosis in every stage can now be combined with the best possible treatment, e.g. pharmaceutical, optical or optoelectrical, or in other words are the basis for preventive, palliative, curative treatment, e.g. using lenses with special design or coating.

According to another aspect of the invention, a test system for measuring at least one of a refractive error or a contrast sensitivity of the visual system of a test person comprises

- a display device arranged for displaying optotype representations according to an embodiment of the invention, the display device comprising an interface configured for receiving optotype representation data; and
- a processing device configured for providing said optotype representations and processing user input, the processing device comprising a first interface connectable to said display device and configured for providing said optotype representations and a second interface configured for receiving user input.

The first and second interfaces may be provided separately or as one bidirectional interface, for example if the display device comprises a touch-screen configured to receive the user input. The described system implements the advantages and characteristics of the claimed method for displaying optotype representations as part of a test system for measuring at least one of a refractive error or a contrast sensitivity of the visual system of a test person. User input may be provided by the test persons themselves or by a supervisor conducting a visual test with the test person using said test system. In one embodiment the display device and the processing device are provided as separate device, e.g. to allow free selection of the distant positioning of the devices. In another embodiment the display device and the processing device are provided in one test apparatus. A test system may comprise additional means for carrying out a subjective refraction test or to measure the contrast sensitivity. It may be provided for monocular and/or binocular tests. It may, for example, be provided as part of a digital visual test system. The processing device may, for example, be provided as or may be comprised in a programmable apparatus.

According to yet another aspect of the invention, a computer program product comprises code portions for executing steps of a method according to an embodiment of the invention as described above when run on a programmable apparatus. The computer program product implements the advantages and characteristics of the claimed method for displaying optotype representations. Depending on the embodiment of the test system, the programmable apparatus may be, may comprise or may be comprised in the processing device of the test system. The invention may, for example, at least partly be implemented in a computer program for running on a computer system, at least including software code portions for performing steps of the method according to the invention when run on a programmable apparatus, such as a computer system, or for enabling the programmable apparatus to perform functions of a device or system according to the invention. The computer program may be provided on a data carrier, such as a CD, DVD, memory card or other storage medium, stored with data loadable in a memory of a computer system, wherein the data represents the computer program. As another example, the data carrier may further be a data connection, such as a telephone cable or a wireless connection. Computer systems may be found in many forms including but not limited to servers, workstations, personal computers, notepads, personal digital assistants, smartphones and various other devices. As an example embodiment, a smartphone may comprise a mobile application using said optotype representations, e.g. radial frequency patterns, for a self-assessment of refractive errors, e.g. habitual refractive errors, or for screening purposes.

While not explicitly described, the present embodiments may be employed in any combination or subcombination.

**Brief description of the drawings**

FIG. 1　　　　schematically illustrates an example of a method for displaying optotype representations to assess at least one of a refractive error or a contrast sensitivity of the visual system of a test person;

FIGs. 2A-2F　　illustrate examples of optotype representations; and

FIG. 3　　　　schematically illustrates an example of a test system for measuring at least one of a refractive error or a contrast sensitivity of the visual system of a test person.

**Description of embodiments**

For a better understanding, the invention will now be explained in more detail in the following description with reference to the drawings. It is understood that the invention is not limited to this exemplary embodiments and that specific features can expediently be combined and/or modified without departing from the scope of the present invention as defined in the appended claims.

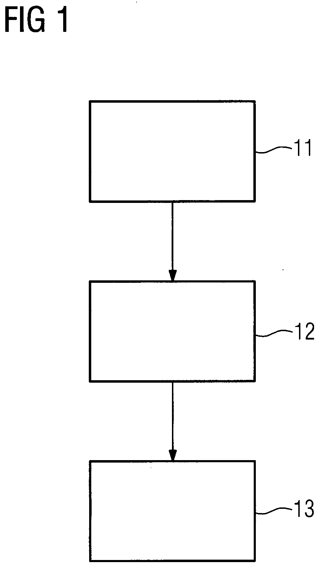

Referring to FIG. 1, a method for displaying optotype representations to assess at least one of a refractive error or a contrast sensitivity of the visual system of a test person according to an aspect of the invention is schematically shown.

In a first step 11 a plurality of optotypes are provided. The plurality of optotypes comprises a base pattern and one or more modulated versions of the base pattern, wherein a contour of the base pattern is defined by a closed curve and the one or more modulated versions of the base pattern have contours being defined by modulated versions of the closed curve. The optotypes may, for example, be received from a storage or calculated by a processing device or represented as printed images on a chart board.

In a second step 12 a test system for measuring at least one of a refractive error or a contrast sensitivity of the visual system of a test person is provided. It should be noted that the first and second step 11, 12 may be carried out either consecutively, the first before or after the second or in parallel, at the same time. The provided test system may, for example, comprise one or more processing devices not only for performing the measurement but also for calculating the optotypes according to parameters useful for the particular test to be carried out and the test person, i.e. the person having his/her visual system tested. For example, for a subjective refraction test, the test system may comprise a set of trial lenses and means for changing the lenses in response to the perception of the presented optotypes by the test person, e.g. a digital phoropter.

In a third step 13 representations of the plurality of optotypes are displayed via a display device of the test system. Depending on the visual test to be carried out the display method may comprise changing parameters of the optotype presentation, such as the presented optotype, a contrast between the optotype presentation and the background, the viewing distance, or the refraction of a lens for correcting a refractive error. In order to stimulate different parts of the visual system, the color of the optotype, its position on the display or a movement of the displayed optotype representation, or an illumination may be changed. In one embodiment, the optotype representation may even be displayed as a three-dimensional object (with or without motion of the object).

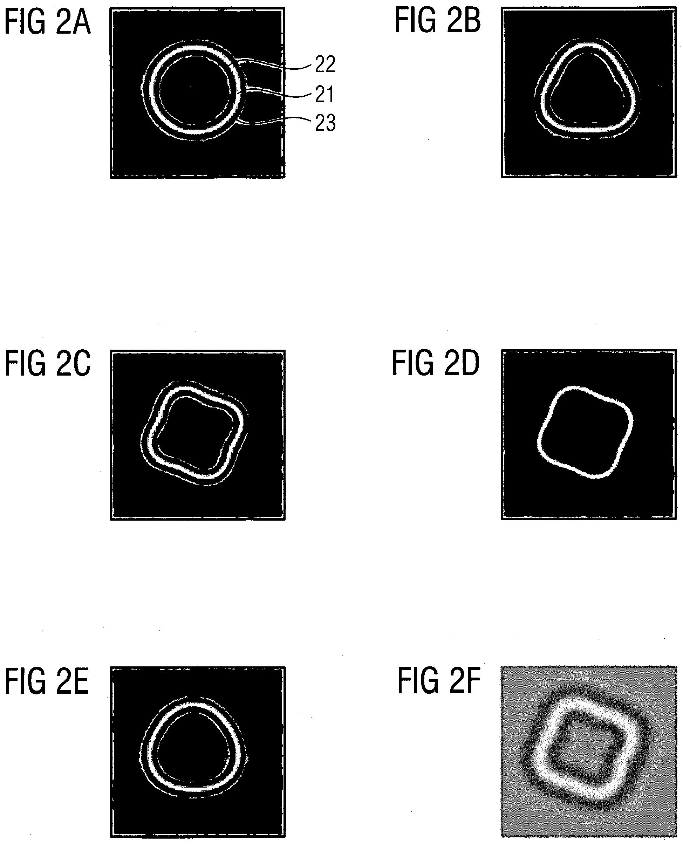

Referring to FIGs. 2A, 2B, 2C, 2D, 2E, and 2F, examples of optotype representations are shown, displayed according to an embodiment of the method described above. More particularly, an example for a circular base pattern and radial frequency (RF) patterns with small modulations on the contour of the circular base pattern, e.g. varied in contrast and spatial frequency, are shown. The examples illustrate at least that a range of spatial parameters can be easily manipulated to allow an optimal refraction or contrast sensitivity estimation.

In FIG. 2A, a non-modulated reference circle is shown as a base pattern on a grey background, where a light region 21 is bordered on an inner side and an outer side by a dark region 22, 23. More particularly, in the shown example the spatial luminance profile (the cross-section of the stimuli) is a 4th derivative of a Gaussian function. Therefore, the shown base pattern as well as the modulated versions shown in FIG. 2B-2F are spatially localized radial frequency (RF) patterns or RF stimuli, and the spatial frequency (visual acuity) can be easily modulated.

In Fig. 2B-2F radially modulated versions of the circle shown in FIG. 2A are illustrated. They are sinusoidally modulated versions of said circle given by the formula $r(\theta) = R_0[1 + A(\theta) \cdot sin(\omega \cdot \theta + \varphi)]$, where $r$ (radius) and $\theta$ (polar angle) are polar coordinates of the radially modulated versions of the circle, i.e. $r$ defines the radius of the modulated or deformed version of the circle at polar angle $\theta$. $R_0$ is the radius of the circle being modulated, i.e. the circle shown in FIG. 2A, $A(\theta)$ is a radial amplitude or spatial modulation amplitude, i.e the amplitude defining the maximum of the radial modulation imposed on the circle. Further, $\omega$ is a radial modulation frequency or radial frequency, and $\varphi$ is a modulation phase, i.e. the angular phase of the modulated version of the circle determining its orientation. The radial amplitude $A(\theta)$ is not permitted to exceed 1.0 (expressed as a proportion of the radius $R_0$) and the radial frequency $\omega$ is always set as an integer value.

By modulating the sinusoidal amplitude of the modulation, different shapes can be represented. In FIG. 2B and FIG. 2C, shapes similar to a triangle and to square, respectively, were generated by changing the radial frequency $\omega$ to different integer values, while the radial amplitude $A(\theta)$ is kept at a value of 1.

While in the other shown Figures, the contrast between the shown pattern and the background is left at 100%, in FIG. 2D the contrast between the shown pattern and the background is reduced in order to demonstrate that the stimulus contrast can vary from 100%.

In FIG. 2E, the radial amplitude or spatial modulation amplitude or shape amplitude $A(\theta)$ is modulated. In the example shown in FIG. 2E, the pattern shown in FIG. 2B is changed by modulating the shape amplitude, which is set to 1 in the other Figures. In FIG. 2E it has been reduced to 0.5 to make the modulated pattern more similar to the reference circle shown in FIG. 2A. The modulated versions can be used to measure shape discrimination thresholds, i.e. the point where it is no longer possible to discriminate between the modulated and non-modulated optotype representations used as stimuli. In FIG. 2F, the spatial frequency has been varied.

To optimally discriminate between variations of these specially shaped patterns the human visual system must integrate all information along the contours. This process demands global integration of the local features such as oriented edges extracted by optical, retinal and cortical early-stage processes.

The spatial resolution and therefore the spatial frequency as well as the visual acuity of such a circle can be modulated externally in order to work on the threshold (smallest angle of resolution) of the test person to be sure to achieve the most natural and advanced correction of refractive errors. In case of measuring the contrast sensitivity, the spatial frequency as well as the contrast are changed. The test can be performed under monocular as well as binocular conditions in order to achieve the best correction under these two viewing conditions.

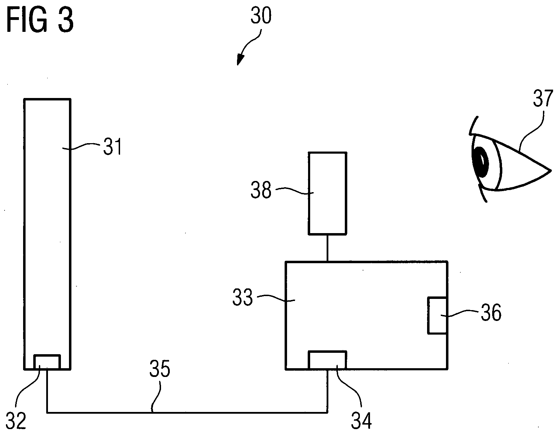

Referring to FIG. 3, an example of a test system for measuring at least one of a refractive error or a contrast sensitivity of the visual system of a test person is schematically illustrated.

The test system 30 comprises a display device 31, e.g. a computer monitor or other display panel, arranged for displaying optotype representations according to an embodiment of the invention. The display device 31 comprises an interface 32 configured for receiving optotype representation data. The test system further comprises a processing device 33 configured for providing the optotype representations to the display device and further configured for processing user input. The processing device 33 comprises a first interface 34 connectable to the display device 31. In the example shown in FIG. 3, the processing device 33 is connected via the first interface 34, data connection 35 and interface 32 to the display device 31. The processing device 33 is configured for providing the optotype representations, i.e. for calculating the optotype representations taking into account parameter values to define the particular optotype and its variations or for reading stored optotype representations from an internal or external storage device (not shown), and for providing the optotype representations to the display device 31. Other parameter values such as the contrast between the optotype representation and the background are either also provided to the display device 31 by the processing device 33 or set directly by the display device 31. The processing device 33 further comprises a second interface 36 configured for receiving user input. The user input may be provided via buttons, a keyboard or as audible or visible commands, depending on the chosen type of user interface as or connected to the second interface 34. User input may be received from a test person, symbolized in FIG. 3 by the schematic contour of an eye 37. However, in a supervised visual test, the supervising person may provide user input in response to information from the test person. Depending on the particular visual test the test system 30 is configured for, it comprises additional means for carrying out the test, as shown by module 38. Module 38 may, for example represent the means, e.g. phoropter means, including one or more lenses, for carrying out a subjective refraction test.

Those skilled in the art will recognize that the boundaries between blocks are merely illustrative and that alternative embodiments may merge blocks or impose an alternative composition of functionality upon various blocks. Unless stated otherwise, terms such as "first" and "second" are used arbitrarily to distinguish between the elements such terms describe. Thus, these terms are not necessarily intended to indicate temporal or other prioritization of such elements. The mere fact that certain features are recited in mutually different claims does not indicate that a combination of these features cannot be used in advantage.

While the principles of the invention have been described above in connection with specific methods and apparatus, it is clearly understood that the description is made only by way of example and not as a limitation of the scope of the invention as defined by the appended claims.

**List of reference signs**

11 Providing a plurality of optotypes
12 Providing a test system
13 Displaying representations of the plurality of optotypes
21 Light region
22 Dark region
23 Dark region
30 Test system
31 Display device
32 Interface of the display device
33 Processing device
34 First interface
35 Data connection
36 Second interface
37 Eye of a test person
38 Additional means module

**Claims**

1. A method for displaying optotype representations to assess at least one of a refractive error or a contrast sensitivity of a visual system of a test person, comprising

- providing (11) a plurality of optotypes, comprising a base pattern and one or more modulated versions of said base pattern, wherein a contour of said base pattern is defined by a closed curve and said one or more modulated versions of said base pattern have contours being defined by modulated versions of said closed curve;
- providing (12) a test system (30) for measuring at least one of a refractive error or a contrast sensitivity of a visual system of a test person;

and

- displaying (13) representations of said plurality of optotypes via a display device (31) of said test system (30).

2. The method as claimed in claim 1, wherein said closed curve is a circle having a radius $R_0$ and said modulated versions of said closed curve are radially modulated versions of said circle.

3. The method as claimed in claim 2, wherein said radially modulated versions are sinusoidally modulated versions of said circle given by

$$r(\theta) = R_0[1 + A(\theta) \cdot sin(\omega \cdot \theta + \varphi)],$$

where $r$ (radius) and $\theta$ (polar angle) are polar coordinates of the radially modulated versions of said circle, $R_0$ is the radius of the modulated circle, $A(\theta)$ is a spatial modulation amplitude, $\omega$ is a radial modulation frequency, and $\varphi$ is a modulation phase.

4. The method as claimed in any of claims 1 to 3, wherein said contour exhibits a cross-sectional luminance profile where a light region (21) is bordered on an inside and on an outside by a dark region (22, 23).

5. The method as claimed in any of claims 1 to 4, wherein said displaying (13) comprises displaying said plurality of optotypes spatially adapted to correspond to a size of a standardized optotype.

6. The method as claimed in any of claims 1 to 5, wherein said displaying (13) comprises changing a contrast between said optotype representations and a background on which said optotype representations are displayed.

7. The method as claimed in any of claims 1 to 6, wherein said displaying (13) comprises at least one of displaying said optotype representations as moving objects or displaying said optotype representations with changing contours over time.

8. The method as claimed in any of claims 1 to 7, wherein displaying (13) said optotype representations comprises displaying one or more of said optotype representations at least partly using one or more colors.

9. Optotype representation, displayed according to a method as claimed in any of claims 1 to 8.

10. Use of an optotype representation as claimed in claim 9 in a test of the visual system of a test person.

11. The use of an optotype representation as claimed in claim 10, wherein said test of the visual system of a test person comprises a subjective refraction test.

12. The use of an optotype representation as claimed in claim 10 or claim 11, wherein said test of the visual system of the test person comprises a contrast sensitivity test.

13. The use of an optotype representation as claimed in claim 12, wherein said test of the visual system further comprises a measurement of a modulation transfer function of the visual system of the test person and a determination of a neural transfer function based on said contrast sensitivity test and said measured modulation transfer function.

14. A test system (30) for measuring at least one of a refractive error or a contrast sensitivity of a visual system of a test person, comprising

- a display device (31) arranged for displaying optotype representations according to claim 9, the display device (31) comprising an interface (32) configured for receiving optotype representation data; and
- a processing device (33) configured for providing said optotype representations and processing user input, the processing device (33) comprising a first interface (34) connectible to said display device (35) and configured for providing said optotype representations and a second interface (36) configured for receiving user input.

15. A computer program product comprising code portions for executing steps of a method as claimed in any of claims 1 to 8 when run on a programmable apparatus.

## FIG 1

```
┌─────────────┐
│             │──11
│             │
└──────┬──────┘
       │
       ▼
┌─────────────┐
│             │──12
│             │
└──────┬──────┘
       │
       ▼
┌─────────────┐
│             │──13
│             │
└─────────────┘
```

FIG 2A

— 22
— 21
— 23

FIG 2B

FIG 2C

FIG 2D

FIG 2E

FIG 2F

FIG 3

Europäisches
Patentamt

European
Patent Office

Office européen
des brevets

## EUROPEAN SEARCH REPORT

Application Number

EP 16 00 2585

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/132304 A1 (VITAL ART AND SCIENCE INC [US]; BARTLETT MICHAEL [US]; WANG YI-ZHONG []) 18 November 2010 (2010-11-18) | 1-12,14, 15 | INV. A61B3/032 A61B3/02 |
| A | * abstract; figures 1a, 2c, 5a-5c * <br> * page 15, line 27 - page 16, line 7 * <br> * page 20, line 30 - page 21, line 5 * <br> * page 22, line 31 - page 23, line 2 * <br> * page 23, lines 11-16 * <br> * page 24, line 1 - page 25, line 8 * | 13 | |
| X | US 2008/186450 A1 (HOLLADAY JACK T [US]) 7 August 2008 (2008-08-07) <br> * abstract; figure 4 * <br> * paragraphs [0008], [0009], [0012], [0031] - [0034] * | 1-12,14, 15 | |
| A,D | Frances Wilkinson ET AL: "Detection and recognition of radial frequency patterns", Vision Research, 1 January 1998 (1998-01-01), pages 3555-3568, XP055377332, DOI: 10.1016/S0042-6989(98)00039-X Retrieved from the Internet: URL:http://ac.els-cdn.com/S004269899800039 X/1-s2.0-S004269899800039X-main.pdf?_tid=6 c062eb2-45d3-11e7-8077-00000aacb360&acdnat =1496216181_6ea42529960ddf94be7c9234f4f53e 3c [retrieved on 2017-05-31] * the whole document * | 1-15 | TECHNICAL FIELDS SEARCHED (IPC) A61B |

The present search report has been drawn up for all claims

| Place of search | Date of completion of the search | Examiner |
|---|---|---|
| Munich | 2 June 2017 | Daniel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 00 2585

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2017

| Patent document cited in search report | | Publication date | Patent family member(s) | | Publication date |
|---|---|---|---|---|---|
| WO 2010132304 | A1 | 18-11-2010 | AU | 2010247901 A1 | 24-11-2011 |
| | | | AU | 2015213323 A1 | 03-09-2015 |
| | | | CA | 2760694 A1 | 18-11-2010 |
| | | | CN | 102458220 A | 16-05-2012 |
| | | | CO | 6460703 A2 | 15-06-2012 |
| | | | EP | 2427095 A1 | 14-03-2012 |
| | | | JP | 5812986 B2 | 17-11-2015 |
| | | | JP | 6049798 B2 | 21-12-2016 |
| | | | JP | 2012525948 A | 25-10-2012 |
| | | | JP | 2015142791 A | 06-08-2015 |
| | | | KR | 20120049850 A | 17-05-2012 |
| | | | KR | 20150068493 A | 19-06-2015 |
| | | | RU | 2011150045 A | 20-06-2013 |
| | | | RU | 2014151374 A | 10-07-2016 |
| | | | SG | 175920 A1 | 29-12-2011 |
| | | | SG | 10201402138P A | 30-07-2014 |
| | | | US | 2012050685 A1 | 01-03-2012 |
| | | | US | 2015374226 A1 | 31-12-2015 |
| | | | US | 2017119245 A1 | 04-05-2017 |
| | | | WO | 2010132304 A1 | 18-11-2010 |
| US 2008186450 | A1 | 07-08-2008 | US | 2005012900 A1 | 20-01-2005 |
| | | | US | 2008186450 A1 | 07-08-2008 |
| | | | US | 2011255056 A1 | 20-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WILKINSON ; FRANCES ; HUGH R. WILSON ; CLAUDINE HABAK.** Detection and recognition of radial frequency patterns. *Vision research,* 1998, vol. 38.22, 3555-3568

- **G. LOFFLER.** Perception of contours and shapes: Low and intermediate stage mechanisms. *Vision Research,* 2008, vol. 48, 2106-2127